# EUROPEAN PATENT SPECIFICATION

(11) **EP 4 112 012 B1**
(45) Date of publication and mention of the grant of the patent: **01.05.2024**
(21) Application number: 20924042.3
(22) Date of filing: 29.10.2020
(51) Int. Cl.: A61F 2/44, A61F 2/30

(54) **INTERVERTEBRAL DISC FUSION CAGE**
ZWISCHENWIRBELFUSIONSKÄFIG
CAGE DE FUSION DE DISQUE INTERVERTÉBRAL

(30) Priority: 09.03.2020 CN 202020281213 U
(43) Date of publication of application: 04.01.2023
(73) Proprietor: Dragon Crown Medical Co., Ltd., Jinan, Shandong 250101 (CN)
(72) Inventor: LI, Weiming, Jinan, Shandong 250101 (CN); YANG, Wenzhou, Jinan, Shandong 250101 (CN); PAN, Huihui, Jinan, Shandong 250101 (CN)
(74) Representative: Sun, Yiming
(86) International application number: PCT/CN2020/124584
(87) International publication number: WO 2021/179628

(56) References cited:
- CN-A- 102 599 994
- CN-A- 106 618 811
- CN-A- 108 113 782
- CN-A- 108 113 782
- CN-U- 203 341 866
- CN-U- 207 506 664
- CN-U- 207 506 664
- US-A1- 2010 331 983
- US-A1- 2010 331 983
- US-A1- 2019 290 446

## Description

### TECHNICAL FIELD

The disclosed device relates to the technical field of medical devices, in particular to an intervertebral disc fusion cage.

### BACKGROUND

An intervertebral disc is divided into nucleus pulposus, which is rich in an elastic jellylike substance, in the central part and annulus fibrosus, which is arranged in concentric circles by multiple layers of fibrocartilage rings, in the peripheral part. The annulus fibrosus in the neck and waist is thick in the front and thin in the back, and the nucleus pulposus is easy to protrude posterolaterally, protruding into a spinal canal or intervertebral foramen and compressing a spinal cord or spinal nerves to form intervertebral disc herniation. In the prior art, intervertebral disc herniation is usually treated by surgically implanting an intervertebral disc fusion cage.

An original shape of the fusion cage is of a 6 mm columnar structure, and the fusion cage is made of metal titanium, has a high degree of biocompatibility and sturdiness and can be implanted in a diseased intervertebral space through a channel with an outer diameter of 7 mm without too much damage to a vertebral body structure. After being in a proper position in the intervertebral space, the fusion cage expands by rotating a handle of a fusion cage installation tool, and its expansion diameter may reach 15 mm to the maximum. By matching with implantation of autologous or allogeneic bones, a height of the intervertebral space is effectively restored and maintained.

Chinese utility model patent CN207506664U discloses an expandable intervertebral fusion cage. By designing an expansion joint with a horizontally arranged contact sheet, and using an expansion sheet to connect the contact sheet with a tubular body of the fusion cage, during a surgical treatment, when the tubular body of the fusion cage is compressed, the expansion sheet bends and expands outward under the action of an external force and drives the contact sheet connected thereto to expand outward at the same time. Since the contact sheet is not directly affected by the external force, the contact sheet may still maintain a horizontal state after compression. Thus, a relatively large contact area may be provided for the vertebral body, the safety during the surgery can be ensured while the height and stability of the intervertebral space are ensured, and an injury to a patient caused by the skew of the expansion joint after compression can be avoided. However, in the expansion process of an actual surgery, due to inconsistency of external pressure and other factors, the expansion degrees of second expansion sheets on both sides of the contact sheet may be inconsistent, and the contact sheet may not be parallel to an axis. With the continuous compression in an axial direction of the fusion cage, the support sheet is bent and deformed, an expansion sequence of the second expansion sheets cannot be determined, and the second expansion sheets may be inclined, thereby causing the expanded contact sheet not to be in a horizontal state, and still causing a ridge-shaped protrusion to be skewed, which makes it difficult to maintain the height of the intervertebral space and the stability of the vertebral body, and still brings an unnecessary harm to the patient.

### SUMMARY

In view of the existing problems in the prior art that it is difficult to grasp an expansion state of a fusion cage implanted in a main body, an expansion order of second expansion sheets cannot be determined, and the second expansion sheets may be inclined, resulting in a skew of a ridge-shaped protrusion, the device provides an intervertebral disc fusion cage, so as to achieve the beneficial effects of ensuring that the intervertebral disc fusion cage expands in sequence and ensuring a contact sheet to be in a horizontal state after expansion.

The device provides an intervertebral disc fusion cage, including a tubular body, where a plurality of support expansion joints which are distributed at intervals and a stabilization expansion joint are arranged on the tubular body in an axial direction and a circumferential direction; each support expansion joint includes a support sheet plate; each support sheet plate is respectively provided with extended support plates at both ends in a length direction; both sides of the bottom of each support sheet plate are respectively connected to the tubular body by means of deformation support sheets; each support sheet plate is provided with an inward-bending stopper for limiting deformation of the top ends of the deformation support sheets; an outward-bending stopper for limiting deformation of the bottom ends of the deformation support sheets is arranged between two adjacent support expansion joints on the tubular body; two oppositely arranged limit blocks are provided between the two adjacent support expansion joints in the circumferential direction of the tubular body; and the two limit blocks abut against each other when the deformation support sheets expand to the maximum.

Further, a connecting portion is provided between the extended support plates and the inward-bending stopper on each support expansion joint; the top ends of the deformation support sheets are connected to the connecting portion; both ends of the inward-bending stopper respectively extend toward the deformation support sheets; and when the deformation support sheets expand to the maximum state, the inner sides of the two oppositely arranged deformation support sheets both abut on the inward-bending stopper.

Further, both ends of each outward-bending stopper respectively extend toward the deformation support sheets; and when the deformation support sheets expand to the maximum state, the outer sides of deformation support sheets abut on the outward-bending stopper.

Further, the stabilization expansion joint includes a plurality of support bars arranged at intervals; a cavity is left between two connected support bars; oppositely arranged stop blocks are arranged at both ends of each cavity; and when the support bars expand to the maximum, the two oppositely arranged stop blocks abut against each other.

Further, a positioning groove is formed in one end, connected to a fusion cage placement tool, of the tubular body.

Further, four support bars are provided and are uniformly arranged in the circumferential direction of the tubular body.

The device has the beneficial effects as follows:
In the intervertebral disc fusion cage provided by the device each support sheet plate is respectively provided with the extended support plates at both ends in the length direction, which increases contact areas with upper and lower end plates and reduces the intensity of pressure on a support surface of the fusion cage, so that the situation that the end plates are broken by the support surface due to small support area and high pressure, resulting in a collapse of an intervertebral space is avoided or reduced. The deformation support sheets are limited by the inward-bending stoppers and the outward-bending stoppers in the expansion and deformation process, which realizes that the support sheet plates are axially parallel to the fusion cage after expansion, and ensures a fusion space of the intervertebral space. The two oppositely arranged limit blocks are provided between two adjacent support expansion joints in the circumferential direction of the tubular body, and when the deformation support sheets expand to the maximum, the two limit blocks abut against each other, so that excessive expansion of the deformation support sheets can be avoided, and the support sheet plates can be ensured to keep parallel to the fusion cage in the axial direction. By changing the size of the support sheet plates, multiple specifications of opening sizes may be obtained, and thus the clinical needs of a variety of sizes can be met. According to the intervertebral disc fusion cage provided by the device the expansion joints on the tubular body can expand in sequence from inside to outside and are all in a state of the same expansion size, which can ensure that the expanded contact sheet is in a horizontal state, avoid the ridge-shaped protrusion from being skewed, and maintain the height of the intervertebral space and the stability of the vertebral body, so as not to bring an unnecessary harm to a patient.

In addition, the device is reliable in design principle and simple in structure, and has a very wide application prospect.

### BRIEF DESCRIPTION OF THE DRAWINGS

In order to more clearly illustrate the embodiments of the device or the technical solutions in the prior art, the accompanying drawings that need to be used in the embodiments or the prior art will be briefly described below. Obviously, a person of ordinary skill in the art may also obtain other drawings based on these drawings without creative effort.
Fig. 1 is a schematic structural diagram of an intervertebral disc fusion cage when being closed according to an embodiment of the utility device.
Fig. 2 is a schematic structural diagram of an intervertebral disc fusion cage after expansion according to an embodiment of the device.
Fig. 3 is a partial structural schematic diagram of an intervertebral disc fusion cage when being closed according to an embodiment of the device.
Fig. 4 is a schematic structural diagram of an intervertebral disc fusion cage in the early stage of expansion according to an embodiment of the device.
Fig. 5 is a partial structural schematic diagram of an intervertebral disc fusion cage after expansion according to an embodiment of the device.

In the drawings, 1, tubular body; 2, support expansion joint; 21, support sheet plate; 22, extended support plate; 23, deformation support sheet; 24, outward-bending stopper; 25, inward-bending stopper; 26, limit block; 3, stabilization expansion joint; 31, support bar; 32, stop block; 4, positioning groove; 5, tubular body inner hole.

### DETAILED DESCRIPTION

In order to make those skilled in the art better understand the technical solutions in the device the technical solutions in the embodiments of the device will be clearly and completely described as below with reference to the accompanying drawings in the embodiments of the device. Obviously, the described embodiments are only a part of, not all of, the embodiments of the device. All other embodiments obtained by a person of ordinary skill in the art based on the embodiments of the device without creative effort shall fall within the protection scope of the utility model.

Key terms appearing in the device are explained below.

An intervertebral disc fusion cage provided by the utility model includes a tubular body 1.

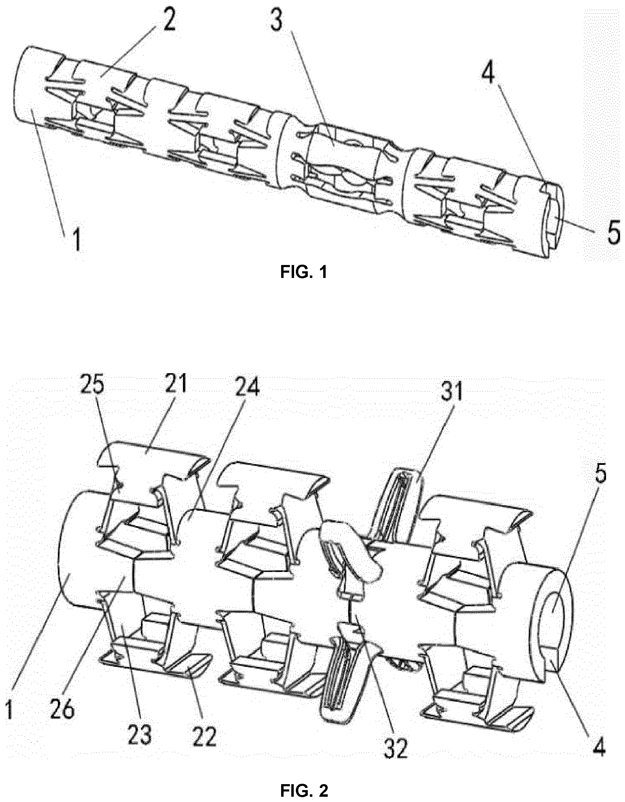

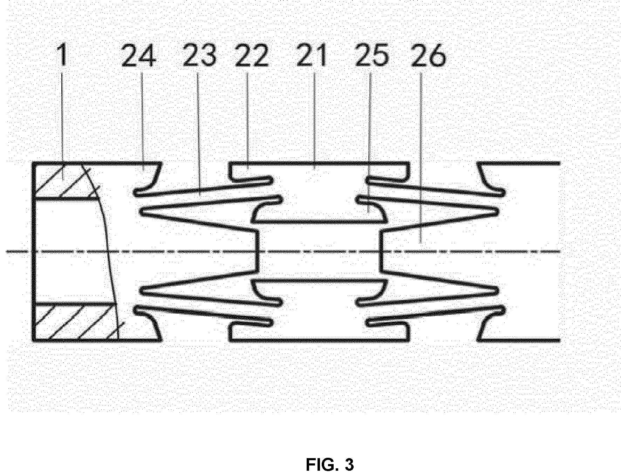

Fig. 1 is a schematic structural diagram of an intervertebral disc fusion cage when being closed according to an embodiment of the device. Fig. 2 is a schematic structural diagram of an intervertebral disc fusion cage after expansion according to an embodiment of the device. Fig. 3 is a partial structural schematic diagram of an intervertebral disc fusion cage when being closed according to an embodiment of the device. As shown in Figs. 1-3, a plurality of support expansion joints 2 which are distributed at intervals and a stabilization expansion joint 3 are arranged on the tubular body 1 in an axial direction and a circumferential direction. After expansion, the expansion joints expand from an initial state shown in Fig. 1 to an expanded state shown in Fig. 2.

Each support expansion joint 2 includes a support sheet plate 21. The support sheet plate 21 is respectively provided with extended support plates 22 at both ends in a length direction, which increases contact areas with upper and lower end plates and reduces the intensity of pressure on a support surface of the fusion cage, so that the situation that the end plates are broken by the support surface due to small support area and high pressure, resulting in a collapse of an intervertebral space is avoided or reduced.

Both sides of the bottom of each support sheet plate 21 are respectively connected to the tubular body 1 by means of deformation support sheets 23. The deformation support sheets 23 are in the shape of a thin sheet, have a certain elasticity and can be bent and deformed in the deformation process.

One end of each deformation support sheet 23 is connected to an inner side of each support sheet plate 21, and the other end of the deformation support sheet 23 is connected to the tubular body 1.

The number of the support expansion joints 2 in the axial direction of the tubular body 1 is set to be at least two, and specifically, may be set according to the needs of a treatment. When more than two support expansion joints 2 are provided, the individual support expansion joints 2 are aligned with each other, that is, the positions of the support sheet plates 21 on the support expansion joints 2 are the same.

Each support sheet plate 21 is provided with an inward-bending stopper 25 for limiting deformation of the top ends of the deformation support sheets 23, and each inward-bending stopper 25 and the corresponding support sheet plate 21 are integrally formed. An outward-bending stopper 24 for limiting deformation of the bottom ends of the deformation support sheets 23 is arranged between two adjacent support expansion joints 2 on the tubular body 1, and the outward-bending stopper 24 and the tubular body 1 are integrally formed.

In the process of expansion and deformation, the deformation support sheets 23 are restricted by the inward-bending stoppers 25 and the outward-bending stoppers 24, so that the support sheet plates 21 after expansion are axially parallel to the fusion cage, and a fusion space of the intervertebral space is ensured.

Two oppositely arranged limit blocks 26 are provided between two adjacent support expansion joints 2 in the circumferential direction of the tubular body 1, and the two limit blocks 26 abut against each other when the deformation support sheets 23 expand to the maximum.

The two limit blocks 26 abut against each other when the deformation support sheets 23 expand to the maximum, so that excessive expansion of the deformation support sheets 23 can be avoided, and the support sheet plates 21 can be ensured to be axially parallel to the fusion cage.

A connecting portion is provided between the extended support plates 22 and the inward-bending stopper 25 on each support expansion joint 2. The top ends of the deformation support sheets 23 are connected to the corresponding connecting portion. Both ends of each inward-bending stopper 25 respectively extend toward the deformation support sheets 23, and both ends of each inward-bending stopper 25 protrude to the outside of the corresponding connecting portion.

When the deformation support sheets 23 expand to the maximum state, the inner sides of the two oppositely arranged deformation support sheets 23 both abut on each inward-bending stopper 25. Each inward-bending stopper 25 is used to limit the deformation amount of the deformation support sheets 23.

Both ends of each outward-bending stopper 24 respectively extend toward the deformation support sheets 23. When the deformation support sheets 23 expand to the maximum state, the outer sides of deformation support sheets 23 abut on the outward-bending stopper 24, which can effectively control the expansion state of the fusion cage.

The stabilization expansion joint 3 includes a plurality of support bars 31 arranged at intervals, and a cavity is left between two connected support bars 31.

In order to keep the expansion state of the fusion cage unchanged, oppositely arranged stop blocks 32 are arranged at both ends of each cavity. When the support bars 31 expand to the maximum, the two oppositely arranged stop blocks 32 abut against each other.

In this embodiment, four support bars 31 are provided and are uniformly arranged in the circumferential direction of the tubular body 1.

A positioning groove 4 is formed in one end, connected to a fusion cage placement tool, of the tubular body 1, and the fusion cage is inserted into the positioning groove 4.

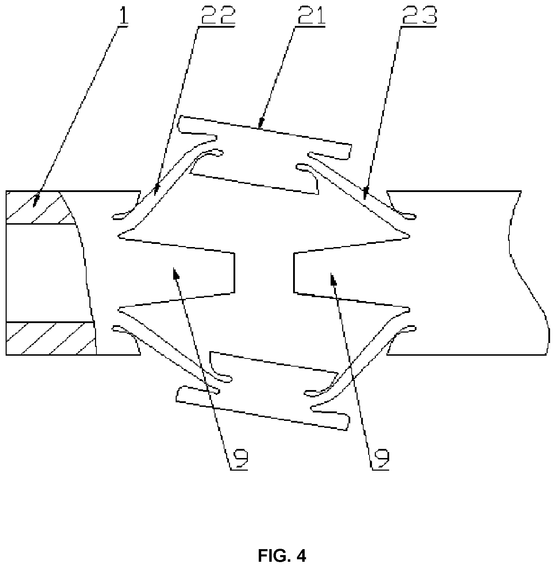

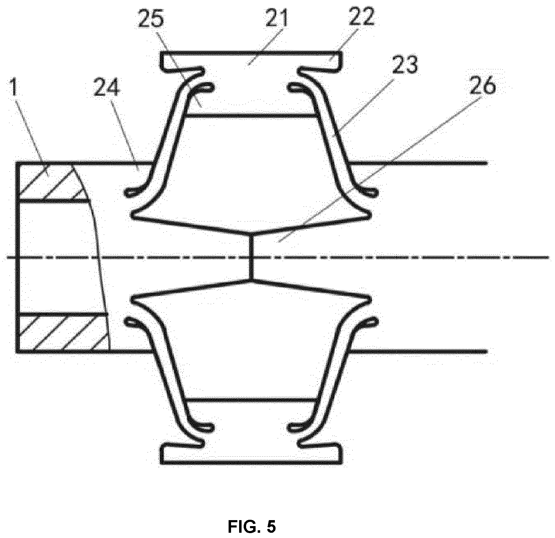

Fig. 4 is a schematic structural diagram of an intervertebral disc fusion cage in the early stage of expansion according to an embodiment of the utility model. Fig. 5 is a partial structural schematic diagram of an intervertebral disc fusion cage after expansion according to an embodiment of the device. As shown in Fig. 4, in the early stage of expansion of the intervertebral disc fusion cage, the extended support plate 22 on one side is deformed firstly. After the fusion cage is continuously compressed, the extended support plate 22 on the other side is deformed. When the deformation support sheets 23 expand to the maximum state, the inner sides of the two oppositely arranged deformation support sheets 23 both abut on the inward-bending stopper 25. When the support bars 31 expand to the maximum, the two oppositely arranged stop blocks 32 abut against each other.

The implementation of this embodiment is that:
A pull rod of an expansion tool penetrates through a tubular body inner hole 5. When the pull rod makes a pull and a pressure in the axial direction, each support plate 21 expands to both sides until the limit blocks 26 on both sides are contacted and pressed tightly, and two support sheet plates 21 expand outward to be combined with and support with the end plates of upper and lower vertebral bodies. Four support bars 31 on the stabilization expansion joints 3 bulge in four oblique directions when expansion and then are bent until the stop blocks 32 are contacted and compressed. In the expansion process, due to inconsistency of external pressure and other factors, there may be cases where expansion of both sides of the support expansion joints 2 is inconsistent, and the support sheet plates 21 are not parallel to the axis of the tubular body 1. With the axial compression of the fusion cage, the support sheet plates 21 are gradually bent and deformed. Under the restriction of the outward-bending stoppers 24 and the inward-bending stoppers 25, after the support sheet plates 21 on one side reach the limit of bending, the support sheet plates 21 on the other side would continue to be bent and deformed until the support sheet plates 21 on the two sides are in a consistent state.

In the intervertebral disc fusion cage provided by the device each support sheet plate 21 is respectively provided with extended support plates 22 at both ends in the length direction, which increases contact areas with the upper and lower end plates and reduces the intensity of the pressure on a support surface of the fusion cage, so that the situation that the end plates are broken by the support surface due to small support area and high pressure, resulting in a collapse of an intervertebral space is avoided or reduced. The deformation support sheets 23 are limited by the inward-bending stoppers 25 and the outward-bending stoppers 24 in the expansion and deformation process, which realizes that the support sheet plates 21 are axially parallel to the fusion cage after expansion, and ensures a fusion space of the intervertebral space. Two oppositely arranged limit blocks 26 are provided between two adjacent support expansion joints 2 in the circumferential direction of the tubular body 1. When the deformation support sheets 23 expand to the maximum, the two limit blocks 26 abut against each other, which can avoid excessive expansion of the deformation support sheets 23, and can ensure that the support sheet plates 21 and the fusion cage remain parallel in the axial direction. By changing the size of the support sheet plates 21, multiple specifications of opening sizes can be obtained to meet the clinical needs of a variety of sizes. According to the intervertebral disc fusion cage provided by the device, the expansion joints on the tubular body can expand in sequence from inside to outside, and are all in a state of the same expansion size, which can ensure that the expanded contact sheet is in a horizontal state, avoid the ridge-shaped protrusion from being skewed, and maintain a height of the intervertebral space and the stability of the vertebral body, so as not to bring an unnecessary harm to a patient.

Although the device has been described in detail with reference to the accompanying drawings and in conjunction with the preferred embodiments, those of ordinary skill in the art can make various equivalent modifications or replacements to the embodiments of the device.

## Claims

1. An intervertebral disc fusion cage, comprising a tubular body (1), wherein a plurality of support expansion joints (2) which are distributed at intervals and a stabilization expansion joint (3) are arranged on the tubular body in an axial direction and a circumferential direction; each support expansion joint (2) comprises a support sheet plate (21); each support sheet plate (21) is respectively provided with extended support plates (22) at both ends in a length direction; both sides of the bottom of each support sheet plate (21) are connected to the tubular body (1) by means of deformation support sheets (23) respectively; each support sheet plate (21) is provided with an inward-bending stopper (25) for limiting deformation of the top ends of the deformation support sheets (23); an outward-bending stopper (24) for limiting deformation of the bottom ends of the deformation support sheets (23) is arranged between two adjacent support expansion joints (2) on the tubular body; two oppositely arranged limit blocks (26) are provided between two adjacent support expansion joints (2) in the circumferential direction of the tubular body (1); and the two limit blocks (26) abut against each other when the deformation support sheets (23) expand to the maximum.

2. The intervertebral disc fusion cage according to claim 1, **characterized in that** a connecting portion is provided between the extended support plates (22) and the inward-bending stopper (25) on each support expansion joint (2); the top ends of the deformation support sheets (23) are connected to the connecting portion; both ends of the inward-bending stopper (25) respectively extend toward the deformation support sheets (23); and when the deformation support sheets (23) expand to the maximum state, the inner sides of the two oppositely arranged deformation support sheets (23) both abut on the inward-bending stopper (25).

3. The intervertebral disc fusion cage according to claim 1, **characterized in that** both ends of each outward-bending stopper (24) respectively extend toward the deformation support sheets (23); and when the deformation support sheets (23) expand to the maximum state, the outer sides of deformation support sheets (23) abut on the outward-bending stopper (24).

4. The intervertebral disc fusion cage according to claim 1, **characterized in that** the stabilization expansion joint (3) comprises a plurality of support bars (31) arranged at intervals; a cavity is left between two connected support bars (31); oppositely arranged stop blocks (32) are arranged at both ends of each cavity; and when the support bars (31) expand to the maximum, the two oppositely arranged stop blocks (32) abut against each other.

5. The intervertebral disc fusion cage according to claim 1, **characterized in that** a positioning groove (4) is formed in one end, connected to a fusion cage placement tool, of the tubular body (1).

6. The intervertebral disc fusion cage according to claim 4, **characterized in that** four support bars (31) are provided and are uniformly arranged in the circumferential direction of the tubular body (1).

## Patentansprüche

1. Bandscheiben-Fusionskäfig, welcher einen röhrenförmigen Körper (1) umfasst, wobei mehrere Stützkompensatoren (2), welche in Intervallen verteilt sind, und ein Stabilisierungskompensator (3) in einer Achsrichtung und einer Umfangsrichtung auf dem röhrenförmigen Körper angeordnet sind; jeder Stützkompensator (2) eine Stützblechplatte (21) umfasst; jede Stützblechplatte (21) in einer Längsrichtung an beiden Enden entsprechend mit verlängerten Stützplatten (22) versehen ist; beide Seiten der Unterseite jeder Stützblechplatte (21) entsprechend mithilfe von Verformungsstützblechen (23) mit dem röhrenförmigen Körper verbunden sind (1); jede Stützblechplatte (21) mit einem nach innen gebogenen Stopper (25) zum Begrenzen einer Verformung der oberen Enden der Verformungsstützbleche (23) versehen ist; ein nach außen gebogener Stopper (24) zum Begrenzen einer Verformung der unteren Enden der Verformungsstützbleche (23) zwischen zwei benachbarten Stützkompensatoren (2) auf dem röhrenförmigen Körper angeordnet ist; in der Umfangsrichtung des röhrenförmigen Körpers (1) zwei gegenüberliegend angeordnete Grenzblöcke (26) zwischen zwei benachbarten Stützkompensatoren (2) vorgesehen sind; und die beiden Grenzblöcke (26) aneinanderstoßen, wenn sich die Verformungsstützbleche (23) auf ein Maximum ausdehnen.

2. Bandscheiben-Fusionskäfig nach Anspruch 1, **dadurch gekennzeichnet, dass** an jedem Stützkompensator (2) ein Verbindungsabschnitt zwischen den verlängerten Stützplatten (22) und dem nach innen gebogener Stopper (25) vorgesehen ist; die oberen Enden der Verformungsstützbleche (23) mit dem Verbindungsabschnitt verbunden sind; sich beide Enden des nach innen gebogenen Stoppers (25) entsprechend hin zu den Verformungsstützblechen (23) erstrecken; und, wenn sich die Verformungsstützbleche (23) auf den Maximalzustand ausdehnen, die Innenseiten der beiden gegenüberliegend angeordneten Verformungsstützbleche (23) beide an den nach innen gebogenen Stopper (25) anstoßen.

3. Bandscheiben-Fusionskäfig nach Anspruch 1, **dadurch gekennzeichnet, dass** sich beide Enden von jedem nach außen gebogenen Stopper (24) entsprechend hin zu den Verformungsstützblechen (23) erstrecken; und, wenn sich die Verformungsstützbleche (23) auf den Maximalzustand ausdehnen, die Außenseiten der Verformungsstützbleche (23) an den nach außen gebogenen Stopper (24) anstoßen.

4. Bandscheiben-Fusionskäfig nach Anspruch 1, **dadurch gekennzeichnet, dass** der Stabilisierungskompensator (3) mehrere Stützstäbe (31) umfasst, die in Intervallen angeordnet sind; zwischen zwei verbundenen Stützstäben (31) ein Hohlraum bleibt; an beiden Enden von jedem Hohlraum gegenüberliegend angeordnete Anschlagblöcke (32) angeordnet sind; und, wenn sich die Stützstäbe (31) auf das Maximum ausdehnen, die beiden gegenüberliegend angeordneten Anschlagblöcke (32) aneinanderstoßen.

5. Bandscheiben-Fusionskäfig nach Anspruch 1, **dadurch gekennzeichnet, dass** in einem Ende des röhrenförmigen Körpers (1), welches mit einem Fusionskäfig-Platzierungswerkzeug verbunden wird, eine Positionierungsnut (4) ausgebildet ist.

6. Bandscheiben-Fusionskäfig nach Anspruch 4, **dadurch gekennzeichnet, dass** vier Stützstäbe (31) vorgesehen sind und in der Umfangsrichtung des röhrenförmigen Körpers (1) gleichmäßig angeordnet sind.

## Revendications

1. Cage de fusion de disque intervertébral, comprenant un corps tubulaire (1), dans laquelle une pluralité de joints de dilatation de support (2) qui sont répartis à intervalles et un joint de dilatation de stabilisation (3) sont agencés sur le corps tubulaire dans une direction axiale et une direction circonférentielle ; chaque joint de dilatation de support (2) comprend une plaque de tôle de support (21) ; chaque plaque de tôle de support (21) est respectivement dotée de plaques de support étendues (22) aux deux extrémités dans une direction longitudinale ; les deux côtés du fond de chaque plaque de tôle de support (21) sont reliés au corps tubulaire (1) au moyen de tôles de support de déformation (23) respectivement ; chaque plaque de tôle de support (21) est munie d'un bloc de butée de flexion vers l'intérieur (25) pour limiter la déformation des extrémités supérieures des tôles de support de déformation (23) ; un bloc de butée de flexion vers l'extérieur (24) pour limiter la déformation des extrémités inférieures des tôles de support de déformation (23) est disposé entre deux joints de dilatation de support adjacents (2) sur le corps tubulaire ; deux blocs de limite (26) disposés de manière opposée sont disposés entre deux joints de dilatation de support (2) adjacents dans la direction circonférentielle du corps tubulaire (1) ; et les deux blocs de limite (26) viennent en butée l'un contre l'autre lorsque les tôles de support de déformation (23) sont déployées au maximum.

2. Cage de fusion de disque intervertébral selon la revendication 1, **caractérisée en ce qu'**une partie de connexion est prévue entre les plaques de support étendues (22) et le bloc de butée de flexion vers l'intérieur (25) sur chaque joint de dilatation de support (2) ; les extrémités supérieures des tôles de support de déformation (23) sont connectées à la partie de connexion ; les deux extrémités du bloc de butée de flexion vers l'intérieur (25) se dilatent respectivement vers les tôles de support de déformation (23) ; et lorsque les tôles de support de déformation (23) se dilatent vers l'état maximal, les côtés intérieurs des deux tôles de support de déformation (23) agencées de manière opposée viennent tous deux en butée sur le bloc de butée de flexion vers l'intérieur (25).

3. Cage de fusion de disque intervertébral selon la revendication 1, **caractérisée en ce que** les deux extrémités de chaque bloc de butée de flexion vers l'extérieur (24) se dilatent respectivement vers les tôles de support de déformation (23) ; et lorsque les tôles de support de déformation (23) se dilatent vers l'état maximal, les côtés extérieurs des tôles de support de déformation (23) viennent en butée sur le bloc de butée de flexion vers l'extérieur (24).

4. Cage de fusion de disque intervertébral selon la revendication 1, **caractérisée en ce que** le joint de dilatation de stabilisation (3) comprend une pluralité de barres de support (31) agencées à intervalles ; une cavité est laissée entre deux barres de support reliées (31) ; des blocs d'arrêt (32) agencés de manière opposée sont agencés aux deux extrémités de chaque cavité ; et lorsque les barres de support (31) se dilatent vers l'état maximal, les deux blocs d'arrêt (32) agencés de manière opposée viennent en butée l'un contre l'autre.

5. Cage de fusion de disque intervertébral selon la revendication 1, **caractérisée en ce qu'**une rainure de positionnement (4) est formée dans une extrémité, reliée à un outil de placement de cage de fusion, du corps tubulaire (1).

6. Cage de fusion de disque intervertébral selon la revendication 4, **caractérisée en ce que** quatre barres de support (31) sont prévues et sont agencées uniformément dans la direction circonférentielle du corps tubulaire (1).
